# EUROPEAN PATENT SPECIFICATION

(11) **EP 1 803 806 B1**
(45) Date of publication and mention of the grant of the patent: **28.10.2009**
(21) Application number: 06252154.7
(22) Date of filing: 20.04.2006
(51) Int. Cl.: C12M 3/00

(54) **Injection apparatus and injection method**
Injektionsvorrichtung sowie Injektionsmethode
Appareil et méthode d'injection

(30) Priority: 28.12.2005 JP 2005380039
(43) Date of publication of application: 04.07.2007
(73) Proprietor: FUJITSU LIMITED, Kawasaki-shi, Kanagawa 211-8588 (JP)
(72) Inventor: ANDO, Moritoshi, Fujitsu Limited, Kawasaki-shi, Kanagawa 211-8588 (JP); YOUOKU, Sachihiro, Fujitsu Limited, Kawasaki-shi, Kanagawa 211-8588 (JP); ITO, Akio, Fujitsu Limited, Kawasaki-shi, Kanagawa 211-8588 (JP)
(74) Representative: Stebbing, Timothy Charles

(56) References cited:
- EP-A- 1 564 575
- WO-A-97/34995
- US-A1- 2003 021 017

## Description

The present invention relates to a technology for improving an efficiency of injecting a substance into a cell in a culture medium using a micro capillary and simplifying a configuration of an injection apparatus.

Recently, research for altering genetic information in a cell is frequently performed by directly injecting genes into a cell. With further advances in research, it is expected that the roles of genes will be clarified, and for example, a tailor-made medication for performing gene therapy suitable for individual genetic characteristics will be possible. Among methods of injecting a gene into a cell, an electrical method (electroporation), a chemical method (lipofection), a biological method (vector method), a mechanical method (microinjection), and an optical method (optoporation) have been proposed. However, the electrical method tends to damage the cell because a cell membrane is broken by letting heavy-current flow in the cell; the chemical method is less efficient because genes to which the method may be introduced are limited; and the biological method cannot confirm safety because all materials cannot be introduced. On the contrary, the mechanical method is highly noted as the safest and most efficient method.

In the mechanical method, as disclosed in, for example, Japanese Patent No. 2553150, an image of a cell magnified by a microscope is captured by a camera, an operator decides a position of a needle called a capillary while observing the captured image displayed on a monitor, and injects the gene by letting the capillary puncture the cell.

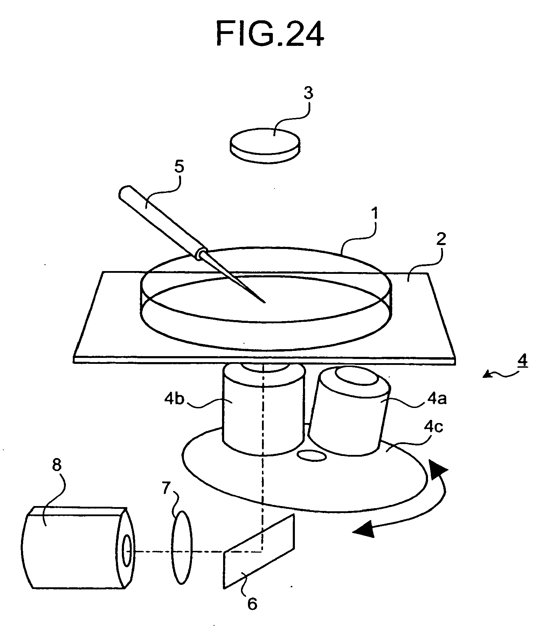

Fig. 24 is a schematic of a conventional microinjection apparatus. In the microinjection apparatus, a dish 1, in which a culture solution with attachment cells is filled, is loaded on a movable stage 2 that is movable in a horizontal direction. An image of cells in the dish 1 onto which light from a light source 3 is directed is magnified by an objective lens 4a or an objective lens 4b mounted on a revolver (turret) 4c of an objective lens unit 4 (the objective lens 4b in the example shown in Fig. 24). The image magnified by the objective lens 4a or the objective lens 4b is reflected towards a camera 8 by a reflector 6, and imaging is made at a position of a lens of the camera 8 by an imaging lens 7.

The magnified image of the cell is captured by the camera 8 and is displayed on a monitor (not shown). The operator moves the movable stage 2 to adjust the position of the dish 1, in order to obtain the magnified image of a desired cell on the monitor, and after determining the position, operates the capillary 5 to inject material such as a gene into the cell.

In such a microinjection apparatus, it is necessary to classify and inject a cell nucleus or a cytoplasm in the cell in response to a purpose of the injection. Therefore, in a cell of size of about several micrometers, because a position of each cell organelle must be accurately confirmed to control the capillary 5, an objective lens of high magnification must be inevitably used. For this reason, in the microinjection apparatus, a plurality of objective lenses 4a, 4b of different magnifications are generally mounted on the revolver 4c, and the cell is designed to be magnified by an objective lens of desired magnification by turning the revolver 4c in a direction of the arrow shown in Fig. 24.

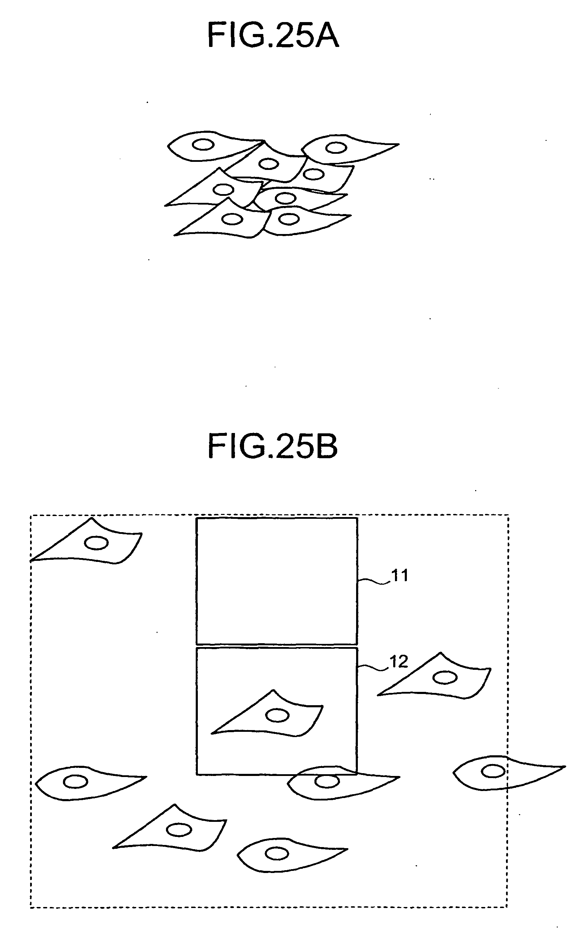

In general, because a dish is much bigger than a cell, when injection is performed, an observation area in which the cell of interest is present must be properly determined within the dish. In this case, in the observation area, it is necessary that a cell density be appropriate to allow each cell to be classified and observed. Namely, it is necessary to search an area of proper cell density, as in a frame 12 shown in Fig. 25B, not an area where cells are densely populated and each cell is overlapped, as shown in Fig. 25A, or an area where no cells are present, as in a frame 11 shown in Fig. 25B. To search the area of proper cell density, because an objective lens of high magnification has a narrow field and its efficiency is poor, it is designed to turn the revolver to change the lens to an objective lens of low magnification and wide field.

The document EP-A-1564575 describes a cell injector device comprising a camera on several lenses to select the desired magnification depending on the image displayed.

However, if injection is performed on a plurality of cells by alternately repeating the search of an appropriate observation area and injection, it is laborious because an objective lens of high magnification and an objective lens of low magnification must be changed each time. As a result, the efficiency of the injection is reduced, resulting in a time-consuming process. In addition, because a plurality of objective lenses of different magnifications is required, a revolver or turret on which the lenses are mounted is also required, leading to a complicated configuration of the unit.

An injection apparatus according to one aspect of the present invention includes a capturing unit that captures an image of a designated position in a culture medium; a first creating unit that creates a wide-field image by synthesizing a plurality of images of the designated position and a periphery of the designated position captured by the capturing unit; a second creating unit that creates a narrow-field image using the image of the designated position captured by the capturing unit; and a displaying unit that arranges and displays the wide-field image and the narrow-field image.

A method of displaying an image including a cell when injecting a substance into the cell in a culture medium using a micro capillary, according to another aspect of the present invention, includes a first capturing step including capturing images of a designated position in the culture medium and images around a periphery of the designated position; a first creating step including creating a wide-field image by synthesizing the images of the designated position and around the periphery of the designated position captured at the first capturing step; a second capturing step including capturing an image of the designated position in the culture medium; a second creating step including creating a narrow-field image using the image of the designated position captured at the second capturing step; and a step of arranging and displaying the wide-field image and the narrow-field image.

The above and other features, advantages and technical and industrial significance of this invention will be better understood by reading the following detailed description of presently preferred embodiments of the invention, when considered in connection with the accompanying drawings, in which:
Fig. 1 is a schematic diagram of an injection apparatus according to a first embodiment of the present invention;
Fig. 2A illustrates a movable stage periphery according to the first embodiment;
Fig. 2B illustrates a dish holder according to the first embodiment;
Fig. 2C illustrates positioning of a dish according to the first embodiment;
Fig. 2D illustrates another configuration of the dish holder according to the first embodiment;
Fig. 3 illustrates a condition of a cell in the dish;
Fig. 4 illustrates a concept of an injection;
Figs. 5A and 5B illustrate a basic operation of the injection;
Fig. 6 is a block diagram of an image processing unit according to the first embodiment;
Fig. 7 is a flowchart of a processing procedure for an operation of the image processing unit according to the first embodiment;
Fig. 8 is a flowchart of a processing procedure for a synthesized-image creation processing according to the first embodiment;
Fig. 9 illustrates a process of creating a synthesized image according to the first embodiment;
Fig. 10 illustrates a change of range of the synthesized image according to the first embodiment;
Fig. 11 illustrates an example of a peripheral edge highlighting in the synthesized image according to the first embodiment;
Fig. 12A illustrates an example of a cell present on a border of an image according to the first embodiment;
Fig. 12B illustrates a processing of the cell present on the border of the image according to the first embodiment;
Fig. 13 is a flowchart of a processing procedure for a center-image creation process according to the first embodiment;
Fig. 14A illustrates a creating operation of a center image according to the first embodiment;
Fig. 14B illustrates a process of creating the center image according to the first embodiment;
Fig. 15 illustrates an example of a display on a monitor according to the first embodiment;
Fig. 16 illustrates an example of a method of designating an injection position according to the first embodiment;
Fig. 17 illustrates an example of marking according to the first embodiment;
Fig. 18 illustrates a process of creating a differential image according to the first embodiment;
Fig. 19 is a block diagram of an image processing unit according to a second embodiment of the present invention;
Fig. 20A illustrates an example of a process of creating a map according to the second embodiment;
Fig. 20B illustrates an example of the map according to the second embodiment;
Fig. 21 illustrates another example of the map according to the second embodiment;
Fig. 22 illustrates still another example of the map according to the second embodiment;
Fig. 23A illustrates a movable range of a cell;
Fig. 23B illustrates still another example of the map according to the second embodiment;
Fig. 24 illustrates an example of a conventional microinjection apparatus;
Fig. 25A illustrates an example of a condition of high cell density; and
Fig. 25B illustrates a cell density suitable for injection.

Exemplary embodiments of the present invention will be explained in detail below with reference to the accompanying drawings.

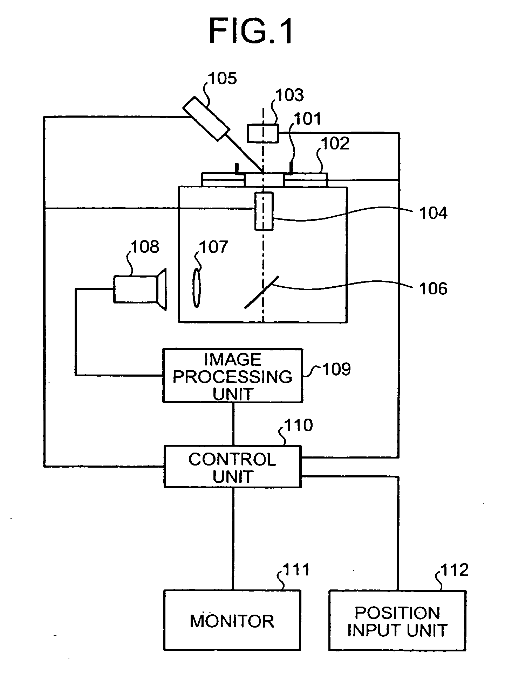

Fig. 1 is a schematic of an injection apparatus according to a first embodiment of the present invention. The injection apparatus includes a movable stage 102 on which a dish 101 is loaded, a light source 103, an objective lens 104, a capillary 105, a reflector 106, an imaging lens 107, a charge-coupled device (CCD) camera 108, an image processing unit 109, a control unit 110, a monitor 111, and a position input unit 112.

The movable stage 102 is mounted so as to be movable in a horizontal direction and adjusts the position of the dish 101 loaded on the upper surface thereof in accordance with the control of the control unit 110. The light source 103 radiates light onto the dish 101 in accordance with the control of the control unit 110 to provide illumination required to observe cells in the dish 101.

The objective lens 104 is a lens of high magnification and narrow-field that magnifies the cells in the dish 101. The magnification of the objective lens 104 is, for example, such that four or five cells are included in the field and each cell or cell organelle may be clearly observed. In addition, the objective lens 104 controls its focus in magnifying the cells in the dish 101 in accordance with the control of the control unit 110.

The capillary 105 is in the form of a needle at the top of the side on the dish 101 and injects a substance such as genetic material into the cell present at the position in the dish 101 input via the position input unit 112 in accordance with the control of the control unit 110. The reflector 106 reflects the magnified image, obtained by magnifying an object by the objective lens 104, in a direction towards the CCD camera 108. The imaging lens 107 images the magnified image obtained by magnifying the object by the objective lens 104 at the position of the lens of the camera 8. The camera 8 captures the magnified image formed by the imaging lens 107 and outputs the captured image to the image processing unit 109.

The image processing unit 109 performs image processing on the image captured by the CCD camera 108, and outputs the obtained image to the control unit 110. Concretely, the image processing unit 109 outputs a synthesized image obtained by connecting a plurality of images and a center image in the synthesized image, creates an image highlighting an edge periphery detected in the cell in the image, and syntheses a plurality of images of different focal depths. The particular image processing performed by the image processing unit 109 will be described in detail later.

The control unit 110 displays the image output from the image processing unit 109 in the monitor 111. In addition, the control unit 110 moves the movable stage 102 in accordance with an instruction from the image processing unit 109, instructs the capillary 105 to perform injection, adjust the focus of the objective lens 104 and controls the on-off state of the light from the light source 103.

The monitor 111 displays the image output from the image processing unit 109 to the control unit 110. In this case, the monitor 111 arranges and displays the synthesized image in which a plurality of images is combined, and the center image located at the center of the synthesized image. The position input unit 112 instructs the control unit 110 to execute the control so as to allow the capturing of the position and the injection to be performed by the designation of the operator upon receipt of the operation of the operator visually observing the monitor 111.

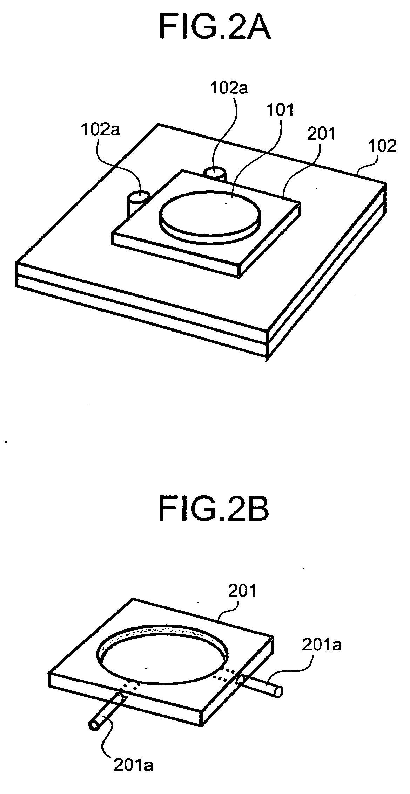

Fig. 2A is a schematic of the dish 101 and the movable stage 102. The dish 101 is fitted into a hole provided at the center of a dish holder 201 and is loaded on the upper surface of the movable stage 102. In this case, by allowing the dish holder 201 to contact with a positioning pin 102a protrusively provided on the upper surface of the movable stage 102 and to be loaded, the position of the dish 101 can be always fixed. Further, a dish presser 201a, which is spring-loaded to press towards the central hole by a spring which is not illustrated, is formed in the dish holder 201, and the position of the dish 101 fitted into the hole can be further securely fixed, as shown in Fig. 2B.

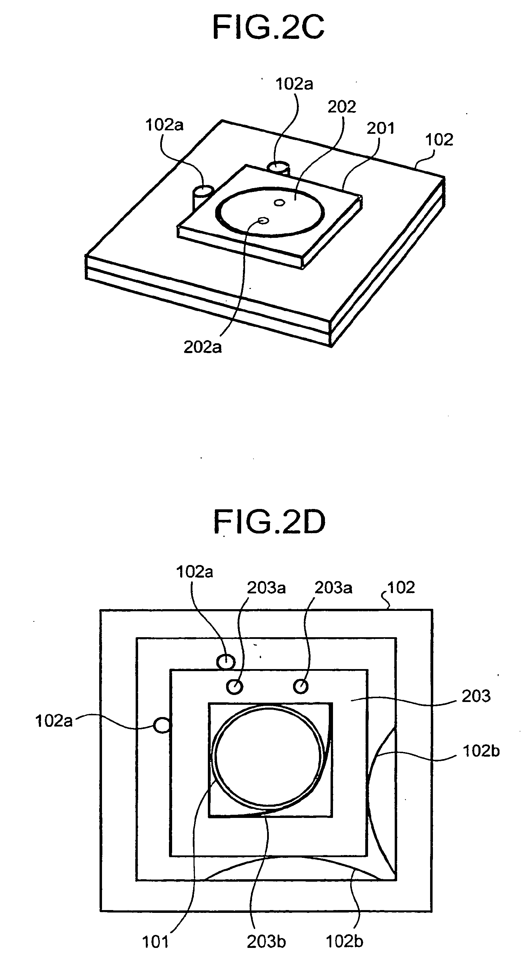

In addition, if the dish 101 is loaded on the movable stage 102 of a different unit, the position of the dish 101 can be always fixed, for example, by disposing a positioning member 202 shown in Fig. 2C at the central hole of the dish holder 201 and adjusting the coordinates of an opening hole 202a protrusively provided at the positioning member 202 so as to allow the coordinates thereof between different units to be reproduced. In addition, by using a dish holder 203 shown in Fig. 2D in place of the dish holder 201, the coordinates of an opening hole 203a provided at the dish holder 203 may be adjusted, thereby dispensing with the positioning member. Further, by providing a pressing spring 102b at the movable stage 102, the dish holder 203 is securely contacted with the positioning pin 102a. In addition, by providing a pressing spring 203b at the dish holder 203 also, the position of the dish 101 in the dish holder 203 is also fixed. For example, a predetermined mark such as a cross mark may be used in place of the opening hole 202a of the positioning member 202 and the opening hole 203a of the dish holder 203.

Thus, because the dish 101 can be always fixed at the same position, even if the dish 101 is moved once to another place after injecting the cell, the position on the movable stage 102 can be easily reproduced to accurately observe the effect.

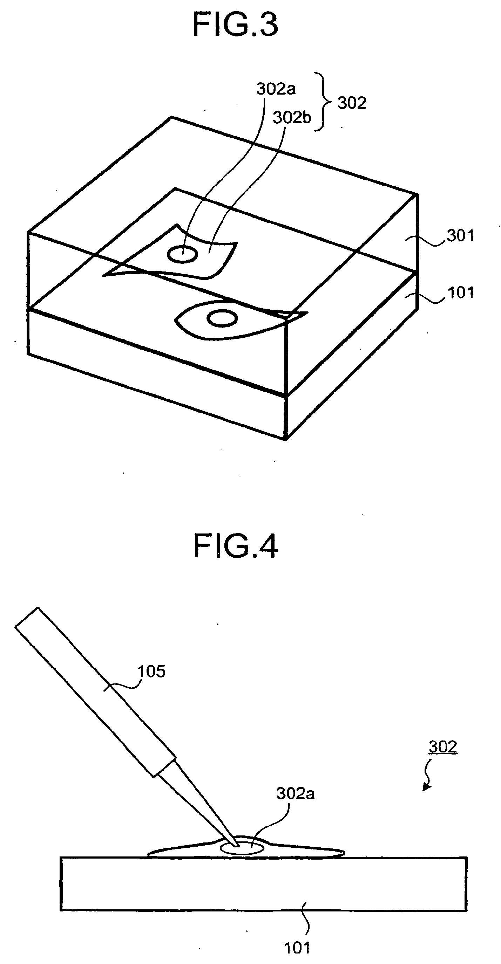

Fig. 3 is a schematic for illustrating a condition of a cell in the dish 101. A culture solution 301 is poured into the dish 101, and if an adherent cell ("attachment cell") is placed into the culture solution 301, a cell 302 adheres to the upper surface of the dish 101 as a certain time elapses. The cell 302 comprises a cell nucleus 302a and a cytoplasm 302b containing various kinds of cell organelles. For example, when substances such as genes are injected into the cell nucleus 302a of the cell 302, as shown in Fig. 4, the top of the capillary 5 punctures the cell nucleus 302a, and the substance is injected into the cell nucleus 302a. In addition, in Fig. 4, the illustration of the culture solution 301 is omitted.

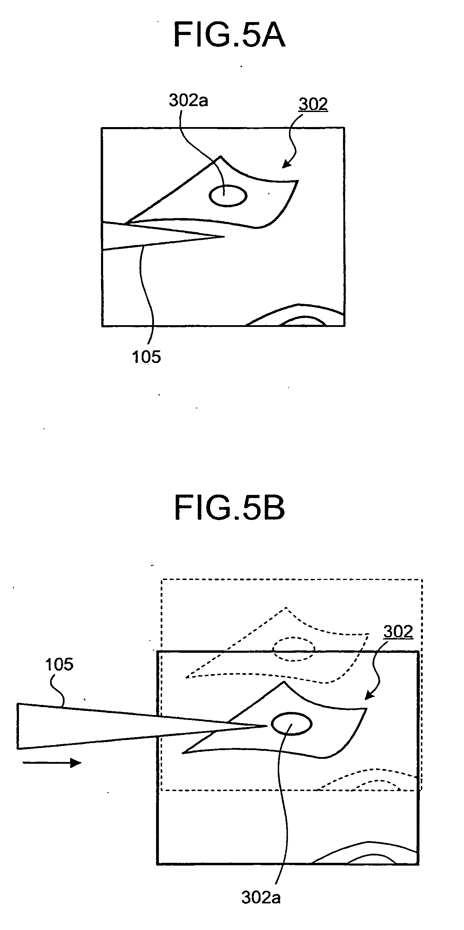

In this case, the magnified image magnified by the objective lens 104 is as shown in, for example, Fig. 5A. Namely, the position of the top of the capillary 5 and that of the cell nucleus 302a of the cell 302 are not aligned. Then, by the operator operating the position input unit 112 to designate the position of the cell nucleus 302a as an injection position, the movable stage 102 is moved by the control of the control unit 110, as shown in Fig. 5B, so that the position of the top of the capillary 5 and that of the cell nucleus 302a are aligned on the same line. Afterwards, the capillary 5 is moved in a direction of the arrow in Fig. 5B to allow the top of the capillary 5 to puncture the cell nucleus 302a. In addition, here, by moving the movable stage 102, the position of the top of the capillary 5 and that of the cell nucleus 302a are designed to be aligned on the same line, however, the capillary 5 may be moved. Thus, in the embodiment, because injection is performed immediately after the position of the injection is designated, it is unnecessary to store the coordinates of the designated injection position.

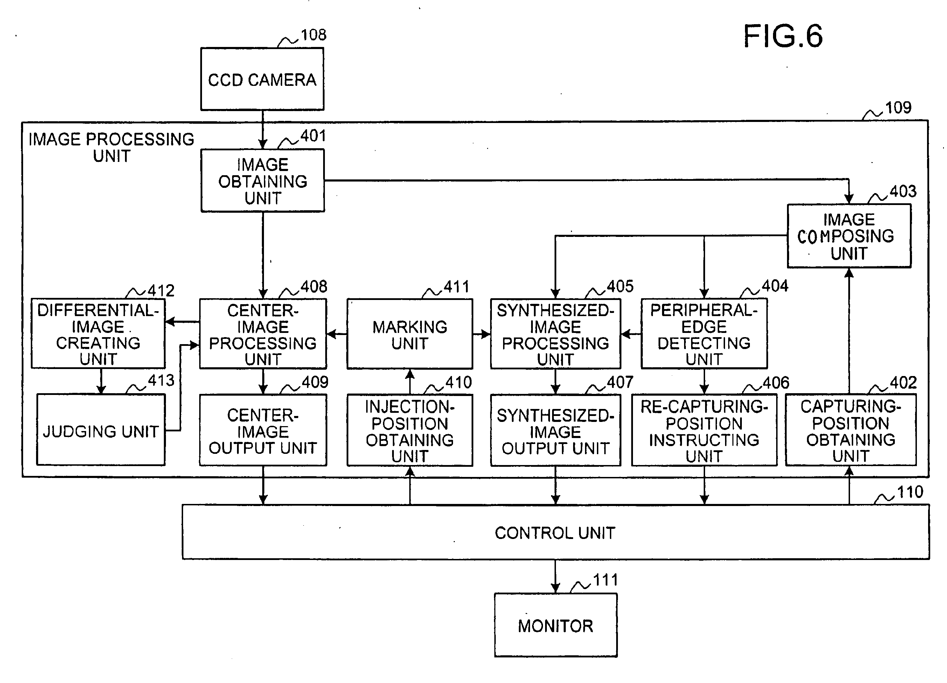

Fig. 6 is a block diagram of the image processing unit 109 according to the first embodiment. The image processing unit 109 includes an image obtaining unit 401, a capturing-position obtaining unit 402, an image composing unit 403, a peripheral-edge detecting unit 404, a synthesized-image processing unit 405, a re-capturing-position instructing unit 406, a synthesized-image output unit 407, a center-image processing unit 408, a center-image output unit 409, an injection-position obtaining unit 410, a marking unit 411, a differential-image creating unit 412, and a judging unit 413.

The image obtaining unit 401 obtains an image captured by the CCD camera 108, and outputs the same to the image composing unit 403 or the center-image processing unit 408. Concretely, the image obtaining unit 401 outputs an observation area search image captured by the CCD camera 108 while the movable stage 102 is being moved within a certain time, and outputs observation images captured by the CCD camera 108, when the movable stage 102 is stopped for a certain time or more, to the center-image processing unit 408.

The capturing-position obtaining unit 402 obtains information on the capturing position of the image captured by the CCD camera 108 from the control unit 110. The image composing unit 403 combines a plurality of images output from the image obtaining unit 401 in accordance with the capturing position obtained by the capturing-position obtaining unit 402, and creates a composite synthesized image for observation area search. In the embodiment, the image composing unit 403 creates the synthesized image by mosaicing a total of nine images where 3 images are arranged vertically and horizontally. However, the number of images processed by the image composing unit 403 can be varied. For example, 25 images where 5 images are arranged vertically and horizontally may be allowed, or the numbers of vertical and horizontal images may not be the same. Thus, by forming a plurality of images into one synthesized image, the conditions such as a peripheral cell density may be grasped.

The peripheral-edge detecting unit 404 performs edge detecting including a differential processing in the synthesized image obtained in the image composing unit 403 to sense the edge periphery of the cell in the synthesized image. The synthesized-image processing unit 405 performs various processes in displaying the synthesized image obtained in the image composing unit 403 in the monitor 111. Concretely, the synthesized-image processing unit 405 highlights the peripheral edges of the cells detected by the peripheral-edge detecting unit 404, for example, by tracing the same in thick line, or the like in the synthesized image. In addition, the synthesized-image processing unit 405 overrides and synthesizes the images re-captured in cell unit with regard to cells located on the borders between each image in the composite synthesized image. Further, the synthesized-image processing unit 405 marks the cells which have been injected in the synthesized image in accordance with the direction of the marking unit 411 to distinguish the already-injected cells from the cells before injection.

The re-capturing-position instructing unit 406 extracts cells of which peripheral edges intersect with the borders between individual images in the synthesized image as a result of the peripheral-edge detecting by the peripheral-edge detecting unit 404, and instructs the control unit 110 to re-capture the extracted cells. Namely, the re-capturing-position instructing unit 406 extracts cells spanning a plurality of images in the synthesized image and instructs the re-capturing of the cell. The control unit 110, after having received this direction, moves the movable stage 102 and instructs the CCD camera 108 to capture the directed position. The synthesized-image output unit 407 outputs the synthesized image to the control unit 110 after the synthesized image is processed by the synthesized-image processing unit 405. The output synthesized image is displayed on the monitor 111 by the control of the control unit 110.

The center-image processing unit 408 performs various processes in displaying the observing image output from the image obtaining unit 401 in the monitor 111. In addition, because the observing image output from the image obtaining unit 401 is an image at a position corresponding to the center image in the synthesized image created by the image composing unit 403, hereinafter, the observing image is called "the center image". Concretely, the center-image processing unit 408 synthesizes two center images of different focuses, and processes the same so as to allow both the cell membrane and cell organelle of the cell to be shown clearly. In this case, the center-image processing unit 408 may perform edge detecting in the center image, or the like, and highlight the peripheral edges of the cell. In addition, the center-image processing unit 408 marks the cells which have been injected in the center image in accordance with the direction of the marking unit 411 to distinguish the cell after injection from the cell before injection. Further, the center-image processing unit 408 makes cells where the effect of injection is apparent, more distinguishable by changing the color of the cell, or the like in accordance with the direction from the judging unit 413.

The center-image output unit 409 outputs the center image after processing to the control unit 110 if the center image is processed by the center-image processing unit 408. The output center image is displayed together with the synthesized image on the monitor 111.

The injection-position obtaining unit 410 obtains the information on the injection designated position input in the position input unit 112, in other words, obtains information on the position of the cell punctured by the capillary 105 to execute injection under control of the control unit 110, from the control unit 110. The marking unit 411 instructs the synthesized-image processing unit 405 and the center-image processing unit 408 to mark the cell where injection is executed in the synthesized image and the center image, respectively. Concretely, the marking unit 411 colors the image frame containing the cell where injection is executed in the synthesized image and colors the peripheral edge of the cell where injection is executed in the center image or adds a predetermined mark to a specific position in the cell.

The differential-image creating unit 412 obtains the image of the cell in the center image before injection, re-obtains the image of the cell at the same position if the center image at the same position is obtained after injection, and creates the differential image of the images of the cell before and after injection. Namely, the differential-image creating unit 412 creates the differential image showing a portion where a change has ocurred before and after injection in the cell. In this case, the differential-image creating unit 412 may find a differential portion between the concentrations (internal details) of the cell before and after injection to create the differential image, or may find a differential portion in shape from a change in profile lines of the cell to create the differential image. The judging unit 413 compares the size of the differential image with a predetermined threshold, judges that injection is properly executed if the size of the differential image is bigger than the predetermined threshold, and conversely judges that injection is not properly executed if the size of the differential image is less than the predetermined threshold. And, the judging unit 413 instructs the center-image processing unit 408 to distinguish the cell where injection is properly executed and the effect of injection is apparent, from other cells.

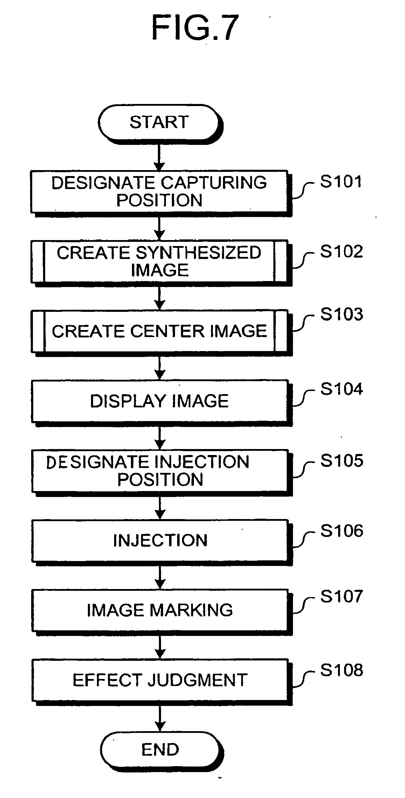

Fig. 7 is a flowchart of a processing procedure for an operation of the image processing unit 109 according to the first embodiment.

The operator operates the position input unit 112 to move the movable stage 102 to a desired position and designates the position in the dish 101 that is captured by the CCD camera 108 (Step S101). When a capturing position is designated, the CCD camera 108 captures the magnified image of the dish 101, and synthesized image creating processing for observation area search is executed (Step S102).

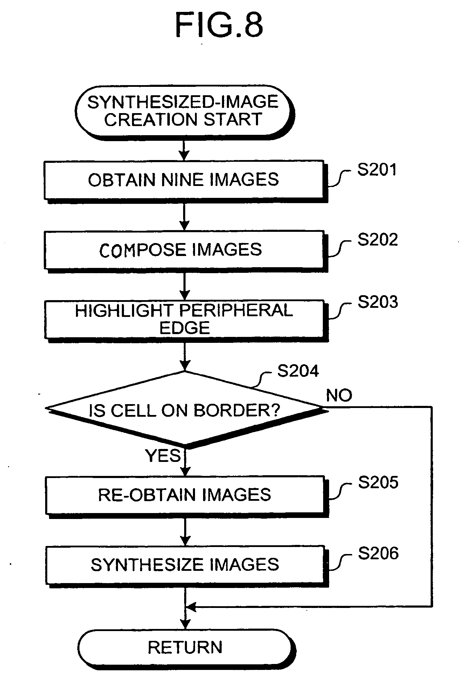

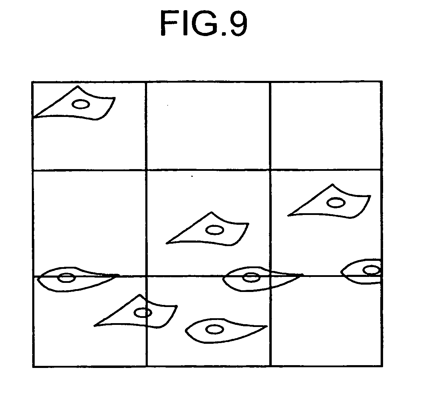

The synthesized image creating processing is executed in accordance with the procedure shown in Fig. 8. For example, as shown in Fig. 9, eight images adjacent to (in the periphery of) the designated capturing position as the center image are sequentially captured (Step S201) to give a total of nine images. In this case, the movement of the movable stage 102 is controlled by the control unit 110, for example, the areas of the upper right image to that of the lower left image in Fig. 9 are sequentially captured images. In addition, the image obtaining unit 401 obtains the captured images and outputs the same to the image composing unit 403. In addition, the capturing-position obtaining unit 402 obtains the capturing positions of each of the nine images from the control unit 110 and outputs the same to the image composing unit 403. The number of captured images to be combined is not limited to nine images, as mentioned above.

The image composing unit 403 pieces together the nine images in response to the capturing positions (Step S202). The obtained synthesized image is held in the synthesized-image processing unit 405, output from the synthesized-image output unit 407, and displayed on the monitor 111 through the control unit 110. In this case, the image size of the synthesized image is suitably contracted so as to be able to be displayed on the monitor 111. Therefore, even if the magnification of the objective lens 104 is high, the substantial magnification of the synthesized image is smaller than that of the objective lens 104.

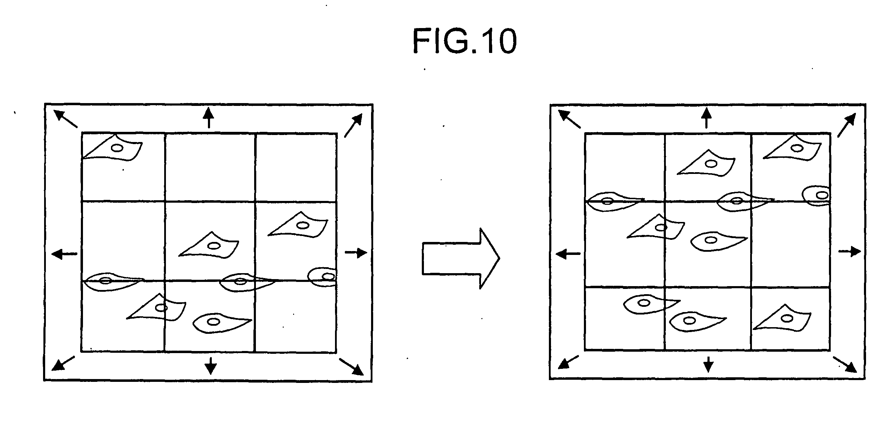

In this case, arrows in eight directions around the synthesized image are shown on the monitor 111, for example, as shown in Fig. 10. The operator may change the scope of (scroll) the synthesized image while observing the monitor 111. Namely, for example, in the left diagram in Fig. 10, if the operator operates the position input unit 112 to designate the lower arrow, the CCD camera 108 obtains the nine images again while the movable stage 102 is being moved again, as shown in Fig. 10, and the area covered by the synthesized image is moved by one image portion downwards.

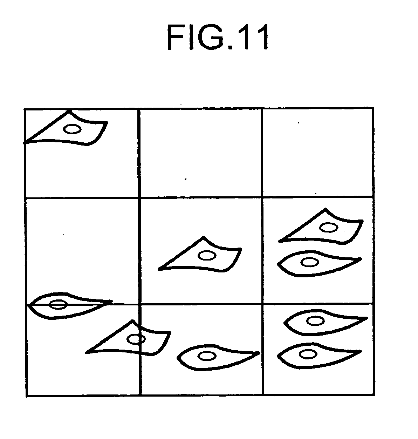

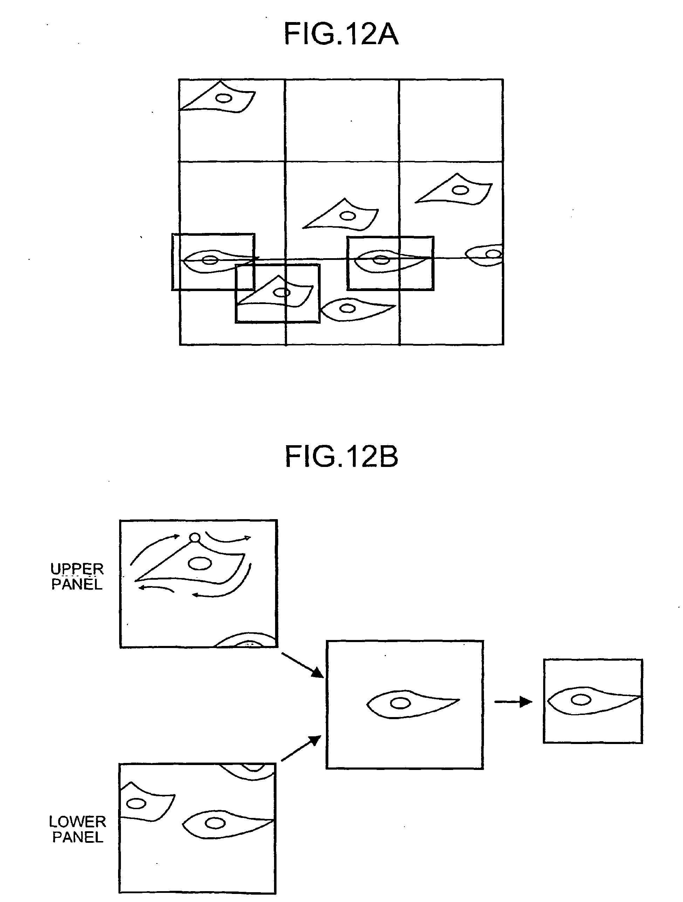

The peripheral-edge detecting unit 404 performs edge detection on the synthesized image, and detects the profile line of the cell in the synthesized image. The detected profile line is highlighted by being overlaid by a thick line, or the like by the synthesized-image processing unit 405, for example, as shown in Fig. 11 (Step S203), and is displayed on the monitor 111 again. Further, if the peripheral-edge detecting unit 404 detects the profile line of the cell, the re-capturing-position instructing unit 406 judges whether a cell displayed crossing the borders of a plurality of images is present, for example, as in the cells enclosed by the black frames in Fig. 12A (Step S204). This judgment is conducted by judging that the cells are present on the borders of a plurality of images when the noted point of the white circle is moved along the profile line of the cell and the noted point intersects with the border of the cell, for example, as illustrated in the diagram in the upper panel of Fig. 12B. As a result, the re-capturing-position instructing unit 406 instructs the control unit 110 to re-capture the positions of the cells when the cells are present on the borders of a plurality of images. In addition, to judge whether the cells are present on the borders of the images, the operator may visually confirm the synthesized image displayed on the monitor 111 and input the positions of the cells present on the borders of the images in the position input unit 112, or the like.

If the control unit 110 receives a direction from the re-capturing-position instructing unit 406 to adjust the position of the movable stage 102, the CCD camera 108 recaptures the cells present on the borders of the images, and the image obtaining unit 401 obtains the obtained image again (Step S205). Namely, in Fig. 12B, the images of the cells present on the borders between the upper panel and the lower panel are obtained again (refer to the diagram in Fig. 12B), and the image composing unit 403 combines the re-obtained images at the capturing positions. Further, after the synthesized-image processing unit 405 cuts off the re-obtained image in the minimum size containing the cell (refer to the right diagram in Fig. 12B), the image is overwritten on the synthesized image retained in the synthesized-image processing unit 405 and is synthesized (Step S206). The synthesized image thus obtained is again displayed on the monitor 111. This allows the cell off-displayed on the border of the images by an error in the movement of the movable stage 102 at the time of obtaining the nine images to be displayed in a correct shape.

The profile lines of the cells are highlighted by the processes at Steps S201 to S206, and the synthesized image where the cells present on the borders of a plurality of images are also displayed on a correct shape is created. Although the synthesized image is not appropriate for viewing micro cell organelles in the cells, it is useful for searching an area of cell density suitable for an observation area.

Referring back to Fig. 7, if the synthesized image creating processing is completed, center image creating processing for observation is performed (Step S103).

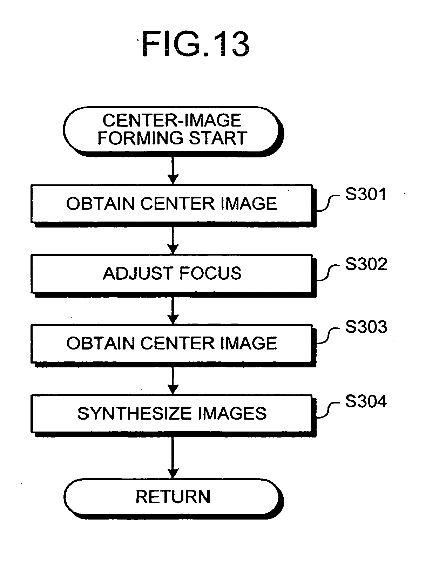

The center-image creation processing is executed in accordance with the procedure in the flowchart shown in Fig. 13. The CCD camera 108 obtains the center image positioned at the center of the synthesized image (Step S301). The center image obtained here is the image of the magnified image where the objective lens 104 focuses the border between the upper surface of the dish 101 and the lower surface of the cell 302.

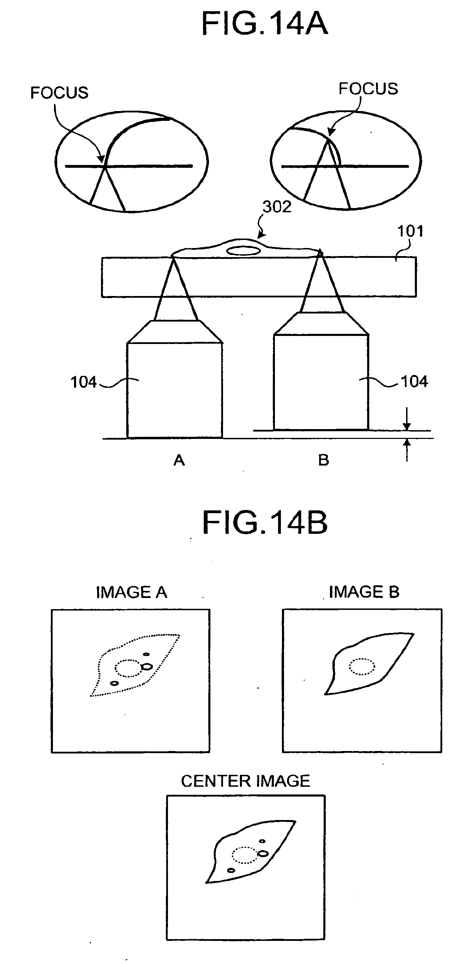

The objective lens 104 is adjusted to focus the cell membrane periphery of the cell 302 in accordance with the control of the control unit 110 as shown in Fig. 14A B (Step S302). After the focus adjustment, the CCD camera 108 obtains the center image again (Step S303).

Although the cell 302 closely contacts the dish 101, it is of a shape whose outer periphery slightly rises from the upper surface of the dish 101, and its thickness is about 5 micrometers. In this case, if the lens 104 focuses on the position shown in Fig. 14A A, for example, as in an image A in Fig. 14B, clear images of the micro cell organelle in the cell attached to the upper surface of the dish 101 are obtained. On the other hand, if the objective lens 104 is adjusted to focus a point about 2 micrometers to 5 micrometers above at the position B shown in Fig. 14A, for example, as in the image B shown in Fig. 14B, a clear image of the cell membrane in the cell 302 is obtained. Then, because the center-image processing unit 408 synthesizes the two images of different focuses (Step S304), a center image where both the profile line of the cell and the cell organelle inside are clear is obtained, as shown in Fig. 14B.

The center image where both the profile line of the cell and the cell organelle inside are clear is created by the processes at Steps S301 to 304. This center image is suitable for observing the micro cell organelles in the cell.

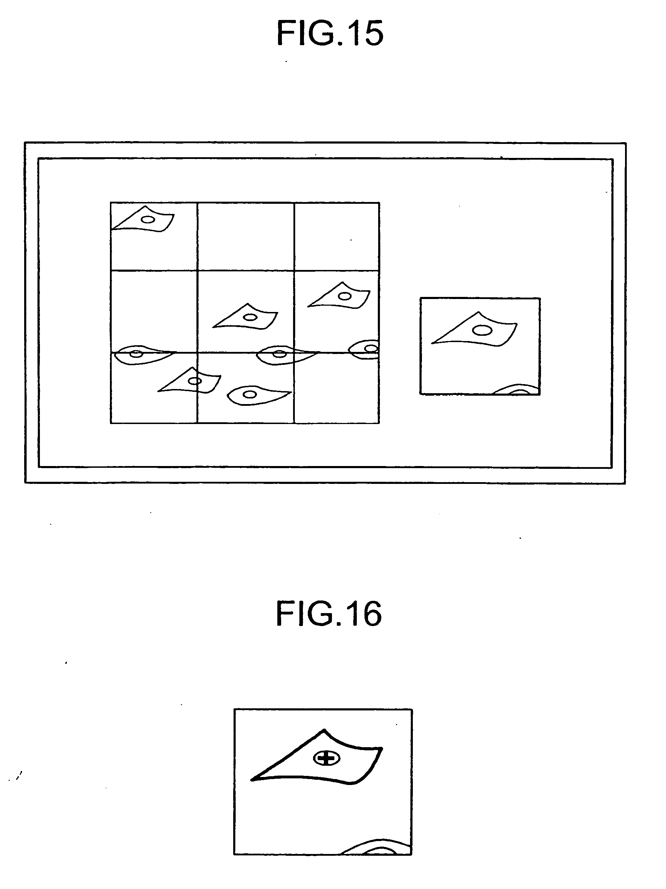

Referring back to Fig. 7, the synthesized image and the center image created by the synthesized image creation processing and the center-image creation processing are arranged and displayed on the monitor 111 as shown in Fig. 15 (Step S104). Even after the synthesized image and the center image are displayed on the monitor 111, if the operator changes the scope of (adjusts the area covered by) the synthesized image, the synthesized-image creation processing per casual Step S102 is performed, accompanied with this procedure, the center-image creation processing at Step S103 is performed. In addition, updating of the center image is always repeated, and the latest status of the cell in the dish 101 is displayed on the monitor 111.

Thus, because the synthesized image and the center image are arranged and displayed on the monitor 111, the operator can monitor a suitable observation area in the center image to perform injection, or view the synthesized image to search for an observation area of suitable cell density. To perform injection, the operator operates the position input unit 112 while viewing the center image in the monitor 111, and designates a position at which injection is desired to be performed in the center image by a cross mark, or the like, for example, as shown in Fig. 16 (Step S105). This designation is notified to the control unit 110, the control unit 110 controls the movable stage 102 and the capillary 5, and injection is performed at the position designated by the operator (Step S106).

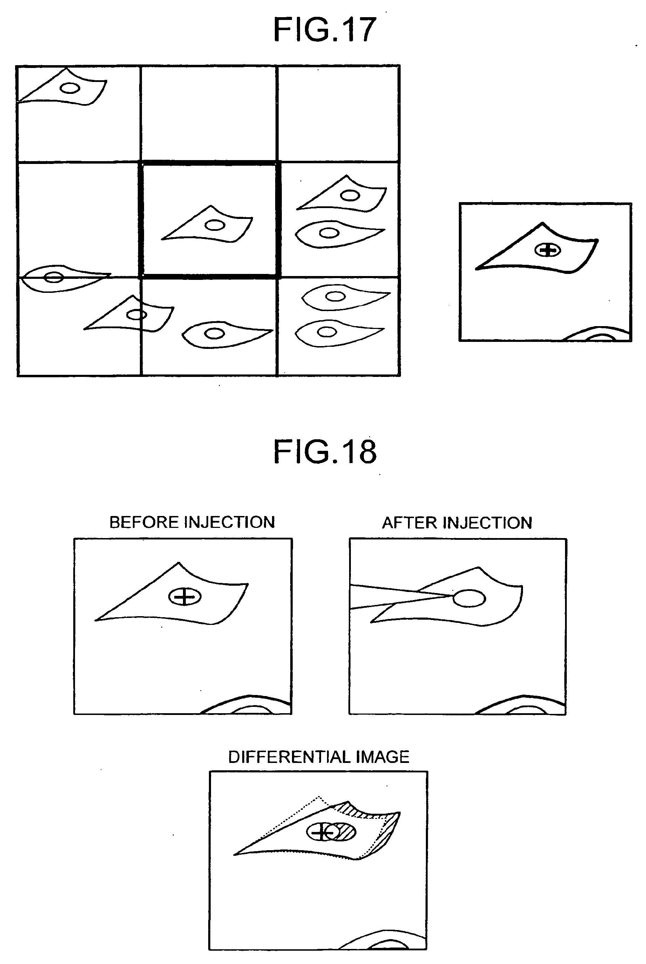

After injection, a mark showing that injection has been performed is added to the synthesized image and the center image displayed on the monitor 111 (Step S107). Namely, the injection-position obtaining unit 410 of the image processing unit 109 obtains information on the position where injection is performed from the control unit 110, and the marking unit 411 marks the synthesized image held in the synthesized-image processing unit 405 and the center image held in the center-image processing unit 408. The marked synthesized image and center image are each output from the synthesized-image output unit 407 and the center-image output unit 409 to the control unit 110, and are displayed on the monitor 111. The marking unit 411 performs marking so as to highlight the image frame containing the injected cell with regard to the synthesized image and to highlight the profile line of the injected cell and the injection position with regard to the center image, for example, as shown in Fig. 17. The marking allows the operator to identify whether injection has been performed on each image frame and each cell.

The search for the observation area by the synthesized image, the designation of the injection position by the center image, and injection, and marking of the injection position are repeated, and injection is performed on a desired number of cells. Once injection has been performed on the desired number of cells, the image processing unit 109 judges the effect of injection (Step, S108). Namely, the differential-image creating unit 412 creates a differential image from the center images before and after injection held in the center-image processing unit 408, and the judging unit 413 judges whether the size of the differential image (the extent of the change) is bigger than a predetermined threshold.

The differential-image creating unit 412 performs the creation of the differential image by finding a difference between the shape of the cell before being injected in the center image and the shape of the cell after being injected in the center image, for example, as shown in Fig. 18. In Fig. 18, the diagonal line portion in the lower drawing is found as a differential image. The judging unit 413 judges whether the area of the diagonal line portion is bigger than a predetermined threshold. It is judged that the injection is properly performed and the effect is apparent if the above area is bigger than the predetermined threshold. The judgment result is notified to the center-image processing unit 408, and processing such as coloring is performed on the image of the cell where the effect of the injection is apparent so as to distinguish the same from other cells. The center-image output unit 409 outputs the center image to the control unit 110, and the image is displayed on the monitor 111. This allows the operator to easily check in which cells the effect of the injection is apparent. In addition, the judgment result by the judging unit 413 is also notified to the synthesized-image processing unit 405, and the image frame containing the cell where the effect of injection is apparent may be colored so as to be able to distinguish the same from other image frames, as in the marking by the marking unit 411.

As described above, according to the first embodiment, the image of the periphery at the designated capturing position is obtained, and the synthesized image obtained by synthesizing the obtained image and the center image disposed at the center of the synthesized image corresponding to the designated capturing position are arranged and displayed on the monitor. Therefore, the observation area suitable for injection can be searched while monitoring the synthesized image that shows the surrounding conditions, and injection can be executed by observing the center image that shows the micro structure of the cell at the same time. Thus, it takes little time to shift the objective lens, or the like, thereby enabling the improvement of the injection efficiency. In addition, because the synthesized image and the center image are created from the image magnified by the same objective lens, a plurality of objective lenses of different magnifications is not required, thereby enabling the simplification of the unit configuration.

A second embodiment of the present invention has the feature that a wider lattice map where an injectable area, an already injected area, a movable area of a cell, or the like is shown is displayed together with the synthesized image and the center image.

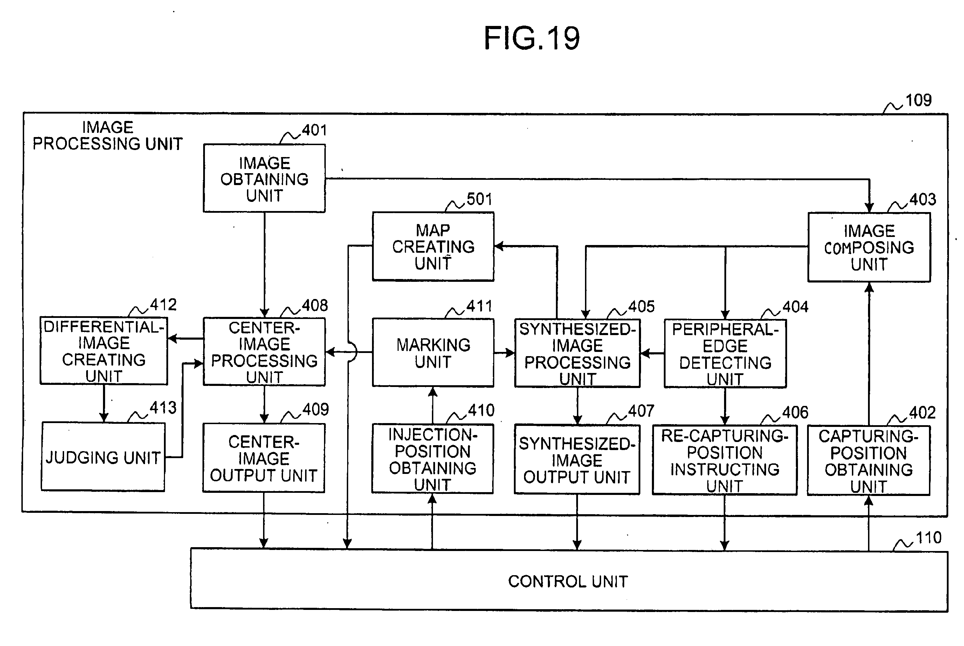

According to the second embodiment, the internal configuration of the image processing unit 109 is different from that of the first embodiment. Fig. 19 is a block diagram of an image processing unit 109 according to the second embodiment. The image processing unit 109 according to the second embodiment has a configuration such that a map creating unit 501 is added to the image processing unit 109 according to the first embodiment.

The map creating unit 501 creates a broader area lattice map where one unit area of the lattice unit area corresponds to one image frame from the synthesized image held in the synthesized-image processing unit 405. One side of one image frame corresponds to, for example, about 100 micrometers, while one side of the whole map corresponds to, for example, about 2 millimeters. The map creating unit 501 shows image frames having a cell density suitable for injection found from the number of cells in each image frame configuring the synthesized image, image frames where injection is completed, and image frames of a scope such that a cell can move, or the like on the map, and displays them in the monitor 111.

According to the second embodiment, for example, at the time of starting the injection apparatus, or the like, the CCD camera 108 sequentially captures the entire area displayed on the map, and a broader image where each image is arranged is created.

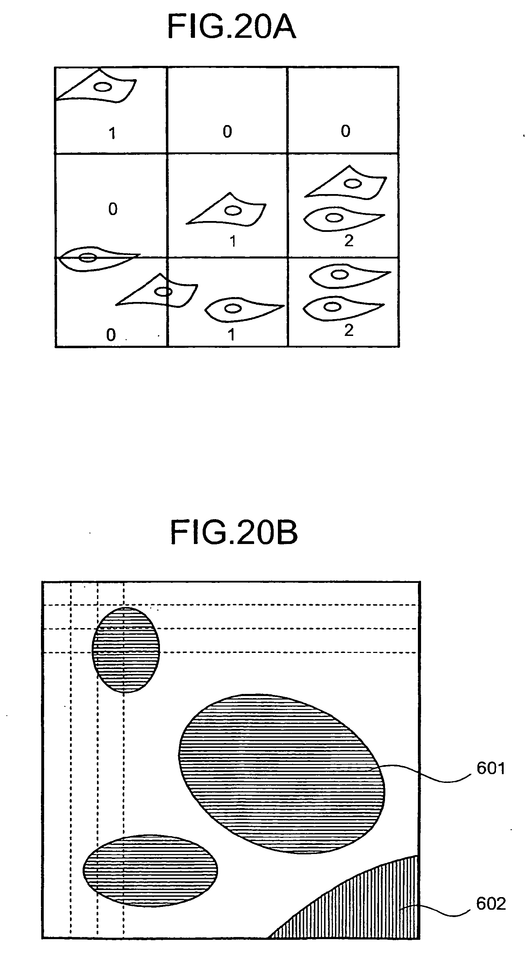

To show whether a cell is suitable for injection on the map, if each image in the broader image is as shown, for example, in Fig. 20A, the map creating unit 501 first counts and records the number of cells in each image frame. In Fig. 20, the numerals in each image frame show the number of cells. In addition, the cells on the borders of the image frames are determined not to be counted to simplify the image processing. And, the map creating unit 501 defines a lattice unit area corresponding to an image frame of cell number of, for example, three to ten to be an area suitable for injection, and a lattice unit area corresponding to an image frame of cell number of, for example, zero to two or eleven or more to be an area not suitable for injection. This is indicated on the map by changing the colors, or the like. One example of this map is shown in Fig. 20B. In Fig. 20B, for example, a scope 601 shown in lateral lines is an area suitable for injection, and a scope 602 marked in vertical lines is an area not suitable for injection.

The operator can roughly decide an objective scope in executing the injection by viewing such a map on the monitor 111 and designating the position where synthesized-image creation processing and center-image creation processing in the first embodiment are performed by operating the position input unit 112.

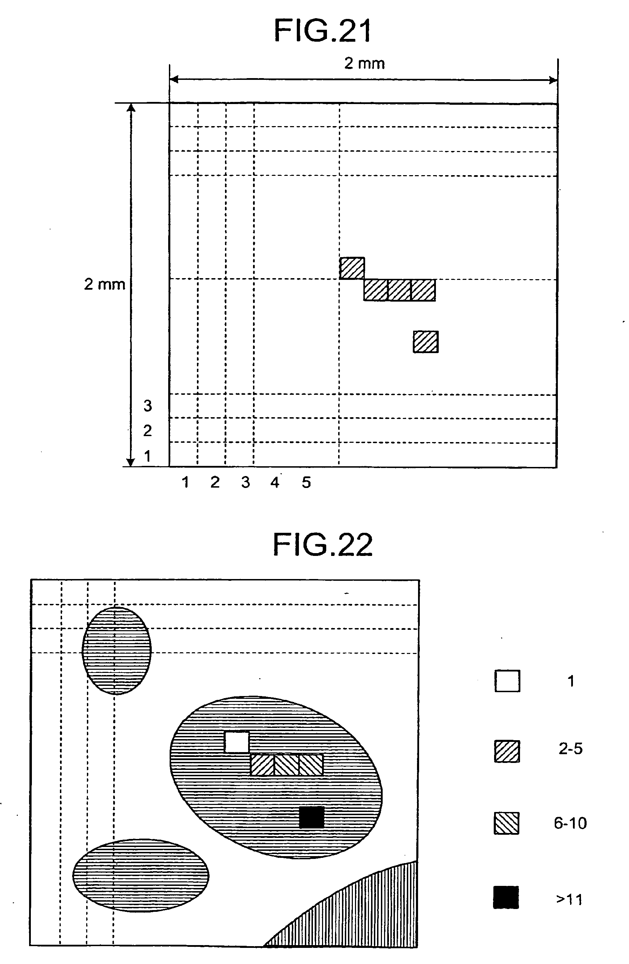

To show an image frame in which injection has been completed on the map, the map creating unit 501 colors a lattice unit area corresponding to the image frame marked on the synthesized image by the marking unit 411 to create the map, for example, shown in Fig. 21. In Fig. 21, the lattice unit area marked with diagonal lines corresponds to the image frame containing the cells where the injection is already completed. This allows the operator to roughly grasp the extent to which injection has been completed.

By counting the number of the cells where injection is performed in the image frame, and by changing the color of the lattice unit area according to the number, the conditions of the injections can be grasped in detail, and areas where the existence or non-existence of the effect should be observed can be studied in detail. At the same time, for example, as shown in Fig. 22, by showing the suitability or non-suitability of injection on the map, the operator can obtain a guideline of areas where injection should be advanced. In such a map, it is unnecessary to count and show the number of cells on all the image frames. After the number of cells on the image frames spaced out at a certain distance is counted, the lattice unit areas may be colored correspondingly to the number of the cells by statistical processing.

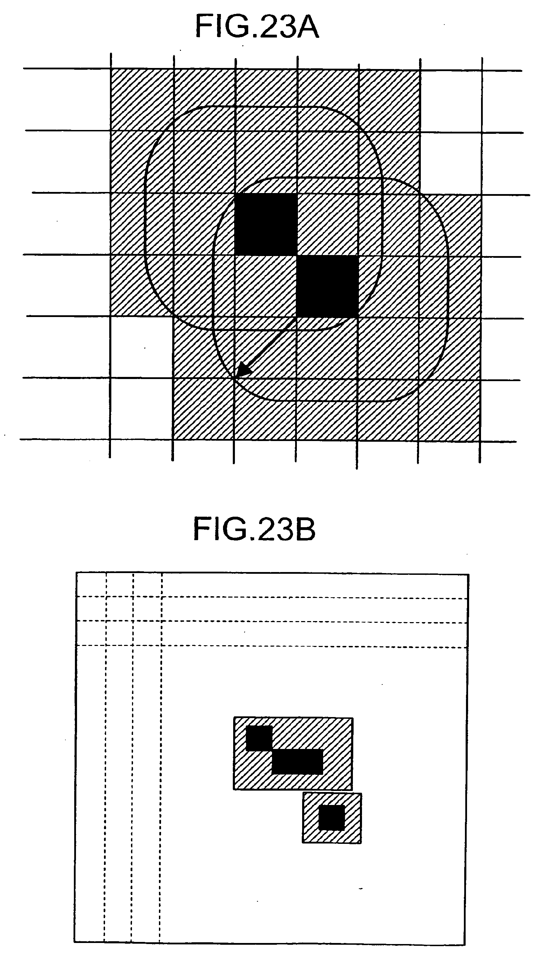

To indicate the image frame of the scope in which the cell is movable on the map, the lattice unit area of the scope corresponding to the degree of activity and mobility speed of the cell may be colored. Here, because the cell may be movable, the cell may not be present even if the same position is observed after the injection. However, because the mobility speed of the cell is limited, for example, as shown in Fig. 23A, the cells present in the lattice unit area shown in black merely move in the approximate distance shown by the arrow in the drawing even at the maximum. Then, if the lattice unit area of the scope shown in the diagonal lines in Fig 23-1 is colored as the movement range of the cell, even when the effect is observed after the injection, the numbers of the cells which are the observation objects are not different from each other before and after the injection, and an efficient observation can be performed. Thus, the example showing the movement range of the cell on the map is shown in Fig. 23A. In Fig. 23B, the black lattice unit area corresponds to the area where the cell was present at the time of injection, and the lattice unit area shown in the diagonal lines corresponds to the movement range (extent of possible movement) of the cells.

As described above, according to the second embodiment, the efficiency of the injection can be further improved because the compatibility or non-compatibility of the injection, the completion status of the injection, and the movement ranges of the cells are shown on a further broader lattice map.

According to the present invention, an operator can search for an observation area of cell density suitable for injection while monitoring the wide-field image and determine a detailed injection position while viewing the narrow-field image, thereby enabling the improvement of the injection efficiency. In addition, the operator can create a wide-field image and a narrow-field image using an image of the same magnification, thereby dispensing with objective lenses of different magnifications to result in a simple unit configuration.

Furthermore, in an embodiment of the present invention, if the position of a substance to be injected and a substance injection position are input in the displayed narrow-field image, because the substance is injected into the substance injection position with a micro capillary just after inputting and a predetermined mark is synthesized at the substance injection position in the narrow-field image, it is unnecessary to store the coordinates of the input substance injection position and the substance injection position can be confirmed in the narrow-field image.

Moreover, in an embodiment of the present invention, because a cell significantly deformed by injection can be classified and distinguished from other cells, the existence or non-existence of the effect (injection) can be easily grasped.

Furthermore, in an embodiment of the present invention, by providing a map such that one lattice unit area corresponds to one narrow-field image, the map being a lattice map corresponding to areas greater than those contained in the wide-field image, and the lattice unit area corresponding to the narrow-field image where the injected cell is present is distinguished from other unit areas, and by displaying such a map with the narrow-field image and the wide-field image, a rough positional relationship between the observation areas corresponding to the wide-field image and the narrow-field image is grasped, and the positions of areas where injection is not completed can be easily confirmed.

## Claims

1. An injection apparatus comprising:
a capturing unit that captures an image of a designated position in a culture medium;
a first creating unit that creates a wide-field image by synthesizing a plurality of images of the designated position and a periphery of the designated position captured by the capturing unit;
a second creating unit that creates a narrow-field image using the image of the designated position captured by the capturing unit; and
a displaying unit that arranges and displays the wide-field image and the narrow-field image.

2. The injection apparatus according to claim 1, further comprising:
an input unit that inputs a position where a substance is injected in the narrow-field image displayed by the displaying unit, wherein
the second creating unit synthesizes a predetermined mark at the position of the narrow-field image input by the input unit.

3. The injection apparatus according to claim 1, further comprising:
an input unit that inputs a position where a substance is to be injected in the narrow-field image displayed by the displaying unit, wherein
the substance is injected into the position using a micro capillary immediately after the position is input by the input unit.

4. The injection apparatus according to claim 1, 2, or 3, wherein
the first creating unit includes a detecting unit that detects a periphery of a cell by edge detecting in the wide-field image; and
a processing unit that performs a processing of highlighting the periphery of the cell detected by the detecting unit in the wide-field image.

5. The injection apparatus according to claim 1, 2, 3, or 4, wherein
the second creating unit performs a processing of detecting and highlighting a periphery of a cell by edge detecting in the narrow-field image.

6. The injection apparatus according to any preceding claim, wherein
the second creating unit creates the narrow-field image by synthesizing a plurality of images of the designated position captured by the capturing unit with different focuses.

7. The injection apparatus according to claim 6, wherein
the second creating unit creates the narrow-field image by synthesizing an image captured by the capturing unit with a focus on a surface of a cell and an image captured by the capturing unit with a focus on a position deviated by 2 micrometers to 5 micrometers above an upper surface of a dish.

8. The injection apparatus according to any preceding claim, wherein
the first creating unit includes
a detecting unit that detects a periphery of a cell by edge detecting in the wide-field image;
an instructing unit that instructs, when the periphery of the cell detected by the detecting unit intersects with a border of the images forming the wide-filed image, a position of the cell as a re-capturing position to the capturing unit; and
a processing unit that overwrites the re-capturing position of the wide-field image with images re-captured by the capturing unit, and synthesizes the overwritten images.

9. The injection apparatus according to claim 8, wherein
the processing unit cuts off a minimum area including the cell from the image re-captured by the capturing unit before overwriting and synthesizing the re-capturing position of the wide-field image.

10. The injection apparatus according to claim 1, further comprising:
an input unit that inputs a position where a substance is injected in the narrow-field image displayed by the displaying unit, wherein
the second creating unit performs a processing of highlighting a periphery of a cell located at the position of the narrow-field image input by the input unit.

11. The injection apparatus according to claim 1, further comprising:
an input unit that inputs a position where a substance is injected in the narrow-field image displayed by the displaying unit, wherein
the first creating unit performs, upon the input unit inputting the position, a processing of highlighting an image frame corresponding to the narrow-field image displayed by the displaying unit in the wide-field image.

12. The injection apparatus according to any preceding claim, further comprising:
a third creating unit that creates a differential image of the narrow-field image before and after the substance is injected; and
a judging unit that judges whether a size of the differential image is larger than a predetermined threshold, wherein
the second creating unit performs a processing of highlighting a cell in the narrow-field image with the size of the differential image larger than the predetermined threshold as a cell having an effect of a substance, based on a result of judgment by the judging unit.

13. The injection apparatus according to any preceding claim, wherein
the displaying unit displays a predetermined symbol inducing a change of the designated position to a periphery of the wide-field image, and
the capturing unit captures the image of the designated position designated in response to the predetermined symbol displayed by the displaying unit.

14. The injection apparatus according to any preceding claim, further comprising:
a fourth creating unit that creates a map in a lattice shape of which one unit area corresponds to one narrow-field image, and covers an area larger than areas included in the wide-field image, wherein
the displaying unit displays the map created by the fourth creating unit together with the wide-field image and the narrow-field image.

15. The injection apparatus according to claim 14, wherein
the fourth creating unit creates the map by distinguishing unit areas corresponding to each narrow-field image according to number of the cells present in the narrow-field image.

16. The injection apparatus according to claim 14 or 15, wherein
the fourth creating unit creates the map by distinguishing a unit area corresponding to the narrow-field image where a substance-injected cell is present from other unit areas.

17. The injection apparatus according to claim 14, 15, or 16, wherein
the fourth creating unit creates the map by distinguishing a lattice unit area corresponding to an area where a cell present in the narrow-field image is movable from other unit areas.

18. The injection apparatus according to any preceding claim, further comprising:
an adjusting unit that adjusts a position of a container that contains the culture medium.

19. The injection apparatus according to claim 18, wherein
the adjusting unit includes
a holding member that fixes and holds the container; and
a projection that makes a contact with the holding member, and determines a position of the holding member.

20. The injection apparatus according to claim 19, wherein
the adjusting unit further includes a position reference member on which a predetermined mark indicating a reference of the position of the holding member is formed, and fixes a position of the predetermined mark.

21. The injection apparatus according to claim 19, wherein
the holding member includes a position reference member on which a predetermined mark indicating a reference of the position of the holding member is formed, and fixes a position of the predetermined mark.

22. A method of displaying an image including a cell when injecting a substance into the cell in a culture medium using a micro capillary, the method comprising:
a first capturing step, including capturing images of a designated position in the culture medium and a periphery of the designated position;
a first creating step, including creating a wide-field image by synthesizing the images of the designated position and the periphery of the designated position captured at the first capturing step;
a second capturing step, including capturing an image of the designated position in the culture medium;
a second creating step, including creating a narrow-field image using the image of the designated position captured at the second capturing step; and
a step of arranging and displaying the wide-field image and the narrow-field image.

## Patentansprüche

1. Injektionsvorrichtung mit:
einer Erfassungseinheit, die ein Bild einer bezeichneten Position in einem Kulturmedium erfasst;
einer ersten Erstellungseinheit, die ein Weitfeldbild durch Synthetisieren einer Vielzahl von Bildern der bezeichneten Position und einer Peripherie der bezeichneten Position erstellt, die durch die Erfassungseinheit erfasst wurden;
einer zweiten Erstellungseinheit, die ein Schmalfeldbild unter Verwendung des Bildes der bezeichneten Position erstellt, das durch die Erfassungseinheit erfasst wurde; und
einer Anzeigeeinheit, die das Weitfeldbild und das Schmalfeldbild anordnet und anzeigt.

2. Injektionsvorrichtung nach Anspruch 1, ferner mit:
einer Eingabeeinheit, die eine Position, wo eine Substanz injiziert wird, in dem Schmalfeldbild eingibt, das durch die Anzeigeeinheit angezeigt wird, bei der
die zweite Erstellungseinheit eine vorbestimmte Marke an der Position des Sehmalfeldbildes synthetisiert, die durch die Eingabeeinheit eingegeben wurde.

3. Injektioimvorrichtung nach Anspruch 1, ferner mit:
einer Eingabccinheit, die eine Position, wo eine Substanz zu injizieren ist, in dem Schmalfeldbild eingibt, das durch die Anzeigeeinheit angezeigt wird, bei der
die Substanz an der Position unter Verwendung einer Mikrokapillare injiziert wird, unmittelbar nachdem die Position durch die Eingabeeinheit eingegeben ist.

4. Injektionsvorrichtung nach Anspruch 1, 2 oder 3, bei der
die erste Erstellungseinheit eine Detektionseinheit enthält, die eine Peripherie einer Zelle durch Randdetektion in dem Weitfeldbild detektiert; und
eine Verarbeitungseinheit, die eine Verarbeitung zum Hervorheben der Peripherie der Zelle ausführt, die durch die Detektionseinheit in dem Weitfeldbild detektiert wurde.

5. Injektionsvorrichtung nach Anspruch 1, 2, 3 oder 4, bei der
die zweite Erstellungseinheit eine Verarbeitung zum Detektieren und Hervorheben einer Peripherie einer Zelle durch Randdetektion in dem Schmalfeldbild ausführt.

6. Injektionsvorrichtung nach einem vorhergehenden Anspruch, bei der
die zweite Erstellungseinheit das Schmalfeldbild durch Synthetisieren einer Vielzahl von Bildern der bezeichneten Position erstellt, die durch die Erfassungseinheit mit verschiedenen Fokussen erfasst wurden.

7. Injektionsvorrichtung nach Anspruch 6, bei der
die zweite Erstellungseinheit das Schmalfeldbild erstellt, indem ein Bild, das durch die Erfassungseinheit mit einem Fokus auf einer Oberfläche einer Zelle erfasst wurde, und ein Bild, das durch die Erfassungseinheit mit einem Fokus auf einer Position, die um 2 Mikrometer bis 5 Mikrometer abweicht, über einer oberen Fläche einer Schale erfasst wurde, synthetisiert werden.

8. Injektionsvorrichtung nach einem vorhergehenden Anspruch, bei der
die erste Erstellungseinheit enthält:
eine Detektionseinheit, die eine Peripherie einer Zelle durch Randdetektion in dem Weitfeldbild detektiert;
eine Anweisungseinheit, die dann, wenn die Peripherie der Zelle, die durch die Detektionseinheit detektiert wurde, eine Grenze der Bilder kreuzt, die das Weitfeldbild bilden, eine Position der Zelle als Neuerfassungsposition für die Erfassungseinheit anweist; und
eine Verarbeitungseinheit, die die Neuerfassungsposition des Weitfeldbildes mit Bildern überschreibt, die durch die Erfassungseinheit neu erfasst wurden, und die darübergeschriebenen Bilder synthetisiert.

9. Injektionsvorrichtung nach Anspruch 8, bei der
die Verarbeitungseinheit einen minimalen Bereich mit der Zelle von dem durch die Erfassungseinheit neu erfassten Bild ausschneidet, bevor die Neuerfassungsposition des Weitfeldbildes überschrieben und synthetisiert wird.

10. Injektionsvorrichtung nach Anspruch 1, ferner mit:
einer Eingabeeinheit, die eine Position, wo eine Substanz injiziert wird, in dem Schmalfeldbild eingibt, das durch die Anzeigeeinheit angezeigt wird, bei der
die zweite Erstellungseinheit eine Verarbeitung zum Hervorheben einer Peripherie einer Zelle ausführt, die an der Position des Schmalfeldbildes angeordnet ist, die durch die Eingabeeinheit eingegeben wurde.

11. Injekt-ionsvorrichtung nach Anspruch 1, ferner mit:
einer Eingabeeinheit, die eine Position, wo eine Substanz injiziert wird, in dem Schmalfeldbild eingibt, das durch die Anzeigeeinheit angezeigt wird, bei der
die erste Erstellungseinheit, nachdem die Eingabeeinheit die Position eingegeben hat, eine Verarbeitung zum Hervorheben eines Bildrahmens entsprechend dem Schmalfeldbild, das durch die Anzeigeeinheit angezeigt wird, in dem Weitfeldbild ausführt.

12. Injektionsvorrichtung nach einem vorhcrgchenden Anspruch, ferner mit:
einer dritten Erstellunqseinheit, die ein Differenzbild des Schmalfeldbildes vor und nach dem Injizieren der Substanz erstellt; und
einer Beurteilungseinheit, die beurteilt, ob eine Größe des Differenzbildes größer als eine vorbestimmte Schwelle ist, bei der
die zweite Erstellungseinheit eine Verarbeitung zum Hervorheben einer Zelle in dem Schmalfeldbild bei einer Größe des Differenzbildes, die größer als die vorbestimmte Schwelle ist, als Zelle, die einen Effekt einer Substanz aufweist, auf der Basis eines Resultates der Beurteilung durch die Beurteilungseinheit ausführt.

13. Injektionsvorrichtung nach einem vorhergehenden Anspruch, bei der
die Anzeigeeinheit ein vorbestimmtes Symbol, das eine Veränderung der bezeichneten Position bewirkt, an einer Peripherie des Weitfeldbildes anzeigt und
die Erfassungseinheit das Bild der bezeichneten Position erfasst, die als Antwort auf das vorbestimmte Symbol bezeichnet wird, das durch die Anzeigeeinheit angezeigt wird.

14. Injektionsvorrichtung nach einem vorhergehenden Anspruch, ferner mit:
einer vierten Erstellungseinheit, die eine Abbildung in Gitterform erstellt, von der ein Einheitsbereich einem Schmalfeldbild entspricht und einen Bereich bedeckt, der größer als Bereiche ist, die in dem Weitfeldbild enthalten sind, bei der
die Anzeigeeinheit die Abbildung, die durch die vierte Erstellungseinheit erstellt wird, zusammen mit dem Weitfeldbild und dem Schmalfeldbild anzeigt.

15. Injektionsvorrichtung nach Anspruch 14, bei der
die vierte Erstellungseinheit die Abbildung erstellt, indem Einheitsbereiche entsprechend jedem Schmalfeldbild gemäß der Anzahl der Zellen, die in dem Schmalfeldbild vorhanden sind, unterschieden werden.

16. Injektionsvorrichtung nach Anspruch 14 oder 15, bei der
die vierte Erstellungseinheit die Abbildung erstellt, indem ein Einheitsbereich entsprechend dem Schmalfeldbild, wo eine Zelle mit injizierter Substanz vorhanden ist, von anderen Einheitsbereichen unterschieden wird.

17. Injekrionsvorricrrtung nach Anspruch 14, 15 oder 16, bei der
die vierte Erstellungseinheit die Abbildung erstellt, indem ein Gittereinheitsbereich entsprechend einem Bereich, wo eine in dem Schmalfeldbild vorhandene Zelle beweglich ist, von anderen Einheitsbereichen unterschieden wird.

18. Injektionsvorrichtung nach einem vorhergehenden Anspruch, ferner mit:
einer Einstelleinheit, die eine Position eines Behälters einstellt, der das Kulturmedium enthält.

19. Injektionsvorrichtung nach Anspruch 18, bei der
die Einstelleinheit enthält:
ein Halteglied, das den Behälter fixiert und hält; und
einen Vorsprung, der einen Kontakt mit dem Halteglied herstellt und eine Position des Haltegliedes bestimmt.

20. Injektionsvorrichtung nach Anspruch 19, bei der
die Einstelleinheit ferner ein Positionsreferenzglied enthält, an dem eine vorbestimmte Marke gebildet ist, die eine Referenz der Position des Haltcglicdcs angibt, und eine Position der vorbestimmten Marke fixiert.

21. Injektionsvorrichtung nach Anspruch 19, bei der
das Halteglied ein Positionsreferenzglied enthält, an dem eine vorbestimmte Marke gebildet ist, die eine Referenz der Position des Haltegliedes angibt, und eine Position der vorbestimmten Marke fixiert.

22. Verfahren zum Anzeigen eines Bildes, das eine Zelle enthält, wenn eine Substanz in die Zelle in einem Kulturmedium unter Verwendung einer Mikrokapillare injiziert wird, welches Verfahren umfasst:
einen ersten Erfassungsschritt, der das Erfassen von Bildern einer bezeichneten Position in dem Kulturmedium und einer Peripherie der bezeichneten Position enthält;
einen ersten Erstellungsschritt, der das Erstellen eines Weitfeldbildes durch Synthetisieren der Bilder der bezeichneten Position und der Peripherie der bezeichneten Position enthält, die bei dem ersten Erfassungsschritt erfasst wurden;
einen zweiten Erfassungsschritt, der das Erfassen eines Bildes der bezeichneten Position in dem Kulturmedium enthält;
einen zweiten Erstellungsschritt, der das Erstellen eines Schmalfeldbildes unter Verwendung des Bildes der bezeichneten Position enthält, das bei dem zweiten Erfassungsschritt erfasst wurde; und
einen Schritt zum Anordnen und Anzeigen des Weitfeldbildes und des Schmalteldbildes.

## Revendications

1. Dispositif d'injection comprenant :
une unité d'acquisition qui acquiert une image d'une position désignée dans un milieu de culture ;
une première unité de création qui crée une image de champ large en synthétisant une pluralité d'images de la position désignée et de la périphérie de la position désignée acquises par l'unité d'acquisition ;
une deuxième unité de création qui crée une image de champ étroit en utilisant l'image de la position désignée acquise par l'unité d'acquisition ; et
une unité d'affichage qui agence et affiche l'image de champ large et l'image de champ étroit.

2. Dispositif d'injection selon la revendication 1, comprenant en outre :
une unité d'entrée qui entre une position où est injectée une substance dans l'image de champ étroit affichée par l'unité d'affichage,
dans lequel la deuxième unité de création synthétise une marque prédéterminée au niveau de la position de l'image de champ étroit entrée par l'unité d'entrée.

3. Dispositif d'injection selon la revendication 1, comprenant en outre :
une unité d'entrée qui entre une position où doit être injectée une substance dans l'image de champ étroit affichée par l'unité d'affichage,
dans lequel la substance est injectée dans la position en utilisant un microcapillaire immédiatement après que la position a été entrée par l'unité d'entrée.

4. Dispositif d'injection selon la revendication 1, 2 ou 3,
dans lequel la première unité de création inclut une unité de détection qui détecte la périphérie d'une cellule par détection de contour dans l'image de champ large ; et
une unité de traitement qui effectue un traitement de renforcement lumineux de la périphérie de la cellule détectée par l'unité de détection dans l'image de champ large.

5. Dispositif d'injection selon la revendication 1, 2, 3 ou 4, dans lequel la deuxième unité de création effectue un traitement de détection et de renforcement lumineux de la périphérie d'une cellule par détection de contour dans l'image de champ étroit.

6. Dispositif d'injection selon l'une quelconque des revendications précédentes, dans lequel la deuxième unité de création crée l'image de champ étroit en synthétisant une pluralité d'images de la position désignée acquise par l'unité d'acquisition avec des mises au point différentes.

7. Dispositif d'injection selon la revendication 6, dans lequel la deuxième unité de création crée l'image de champ étroit en synthétisant une image acquise par l'unité d'acquisition avec une mise au point sur la surface d'une cellule et une image acquise par l'unité d'acquisition avec une mise au point sur une position écartée de 2 micromètres à 5 micromètres au-dessus de la surface supérieure d'une coupelle.

8. Dispositif d'injection selon l'une quelconque des revendications précédentes,
dans lequel la première unité de création inclut :
une unité de détection qui détecte la périphérie d'une cellule par détection de contour dans l'image de champ large ;
une unité d'imposition qui impose, lorsque la périphérie de la cellule détectée par l'unité de détection coupe la bordure des images formant l'image de champ large, une position de la cellule comme position de réacquisition pour l'unité d'acquisition ; et
une unité de traitement qui réécrit la position de réacquisition de l'image de champ large avec les images réacquises par l'unité d'acquisition, et qui synthétise les images réécrites.

9. Dispositif d'injection selon la revendication 8, dans lequel l'unité de traitement découpe une zone minimale incluant la cellule de l'image réacquise par l'unité d'acquisition avant de réécrire et de synthétiser la position réacquise de l'image de champ large.

10. Dispositif d'injection selon la revendication 1, comprenant en outre :
une unité d'entrée qui entre une position où une substance est injectée dans l'image de champ étroit affichée par l'unité d'affichage,
dans lequel la deuxième unité de création effectue un traitement de renforcement lumineux de la périphérie d'une cellule située à la position de l'image de champ étroit entrée par l'unité d'entrée.

11. Dispositif d'injection selon la revendication 1, comprenant en outre :
une unité d'entrée qui entre une position où une substance est injectée dans l'image de champ étroit affichée par l'unité d'affichage,
dans lequel la première unité de création effectue, sur l'unité d'entrée entrant la position, un traitement de renforcement lumineux d'une vue d'image correspondant à l'image de champ étroit affichée par l'unité d'affichage dans l'image de champ large.

12. Dispositif d'injection selon l'une quelconque des revendications précédentes, comprenant en outre :
une troisième unité de création qui crée une image différentielle de l'image de champ étroit avant que la substance soit injectée et après qu'elle l'a été ; et
une unité de détermination qui détermine si la taille de l'image différentielle est plus grande qu'un seuil prédéterminé,
dans lequel la deuxième unité de création effectue un traitement de renforcement lumineux d'une cellule de l'image de champ étroit qui a une taille d'image différentielle plus grande que le seuil prédéterminé, en tant que cellule ayant un effet d'une substance, en se basant sur un résultat de détermination par l'unité de détermination.

13. Dispositif d'injection selon l'une quelconque des revendications précédentes,
dans lequel l'unité d'affichage affiche un symbole prédéterminé induisant un remplacement de la position désignée par la périphérie de l'image de champ large, et
dans lequel l'unité d'acquisition acquiert l'image de la position désignée, désignée en réponse au symbole prédéterminé affiché par l'unité d'affichage.

14. Dispositif d'injection selon l'une quelconque des revendications précédentes, comprenant en outre :
une quatrième unité de création qui crée une carte dans une forme de quadrillage dont une zone unitaire correspond à une image de champ étroit, et qui couvre une zone plus grande que les zones incluses dans l'image de champ large,
dans lequel l'unité d'affichage affiche la carte créée par la quatrième unité de création conjointement avec l'image de champ large et l'image de champ étroit.

15. Dispositif d'injection selon la revendication 14, dans lequel la quatrième unité de création crée la carte en distinguant des zones unitaires correspondant à chaque image de champ étroit d'après le nombre de cellules présentes dans l'image de champ étroit.

16. Dispositif d'injection selon la revendication 14 ou 15, dans lequel la quatrième unité de création crée la carte en distinguant, des autres zones unitaires, une zone unitaire correspondant à l'image de champ étroit où est présente une cellule dans laquelle a été injectée une substance.

17. Dispositif d'injection selon la revendication 14, 15 ou 16, dans lequel la quatrième unité de création crée la carte en distinguant, des autres zones unitaires, une zone unitaire de quadrillage correspondant à une zone où une cellule présente dans l'image de champ étroit est mobile.

18. Dispositif d'injection selon l'une quelconque des revendications précédentes, comprenant en outre une unité d'ajustement qui ajuste la position d'un récipient qui contient le milieu de culture.

19. Dispositif d'injection selon la revendication 18, dans lequel l'unité d'ajustement inclut :
un organe de maintien qui fixe et maintient le récipient ; et
une protubérance qui entre en contact avec l'organe de maintien, et qui détermine la position de l'organe de maintien.

20. Dispositif d'injection selon la revendication 19, dans lequel l'unité d'ajustement inclut en outre un organe de référence de position sur lequel est formée une marque prédéterminée indiquant une référence de la position de l'organe de maintien, et qui fixe la position de la marque prédéterminée.

21. Dispositif d'injection selon la revendication 19, dans lequel l'organe de maintien inclut un organe de référence de position sur lequel est formée une marque prédéterminée indiquant une référence de la position de l'organe de maintien, et qui fixe la position de la marque prédéterminée.

22. Procédé d'affichage d'une image incluant une cellule lors de l'injection d'une substance dans la cellule dans un milieu de culture en utilisant un microcapillaire, le procédé comprenant :
une première étape d'acquisition, incluant l'acquisition d'images d'une position désignée dans le milieu de culture et de la périphérie de la position désignée ;
une première étape de création incluant la création d'une image de champ large en synthétisant les images de la position désignée et de la périphérie de la position désignée acquises à la première étape d'acquisition ;
une seconde étape d'acquisition, incluant l'acquisition d'une image de la position désignée dans le milieu de culture ;
une seconde étape de création, incluant la création d'une image de champ étroit en utilisant l'image de la position désignée acquise à la seconde étape d'acquisition ; et
une étape d'agencement et d'affichage de l'image de champ large et de l'image de champ étroit.
